# EUROPEAN PATENT APPLICATION

(11) **EP 1 652 932 A1**
(43) Date of publication of application: **03.05.2006**
(21) Application number: 04025919.4
(22) Date of filing: 02.11.2004
(51) Int. Cl.: C12N 15/867, C12N 15/90, C12N 5/10, C12N 7/01, A61K 48/00

(54) **Method for the generation of virus producing cell lines and cell lines**

(71) Applicant: GBF Gesellschaft für Biotechnologische Forschung mbH, 38124 Braunschweig (DE)
(72) Inventor: Wirth, Dagmar, 38124 Braunschweig (DE); Hauser, Hansjörg, 38302 Wolfenbüttel (DE); Schucht, Roland, 38126 Braunschweig (DE)
(74) Representative: Kröncke, Rolf

(57) **Abstract**

The present invention relates to a method for generating virus producing cell lines. In particular, the present invention relates to methods allowing the production of virus producing cell lines without conducting laborious selection work. Moreover, the present invention pertains to virus producing cell lines obtained with the methods according to the present invention as well as to virus obtained using said cell lines.

## Description

The present invention relates to a method for generating virus producing cell lines. In particular, the present invention relates to methods allowing the production of virus producing cell lines without conducting laborious selection work. Moreover, the present invention pertains to virus producing cell lines obtained with the methods according to the present invention as well as to virus obtained using said cell lines.

### Background of the invention

Recent successes in gene therapy clinical trials have demonstrated the high potential of the use of gene therapy to cure and slow down a wide scope of diseases. However, the efficient introduction of therapeutic genes into human cells or tissues remains the main problem to be solved.

Various methods have been suggested for introducing the therapeutic nucleotides or genes into the cells or tissue. These techniques include the use of viral vectors. Generally, viral helper genes (the so-called helper functions) are expressed in cells to specifically package into virus particles and transduce the therapeutic gene of interest. Adenoviral vectors are frequently used since the particles are robust, can be easily produced in high titers and efficiently infect a broad spectrum of cells. Retroviruses, in particular the genera of mammalian C type retroviruses and lentiviruses are the most widely used for stable gene transfer and expression of the gene product, Highly sophisticated vector systems have been developed for specialised purposes. Most prior art knowledge is based mainly on mammalian C type viruses (also called retroviruses). Accordingly, most gene therapy trials are based on this virus transfer system. The recombinant retroviral gene delivery methods which have thus far been most extensively used in view of the following advantages: i.) the efficient entry of genetic material into cells; ii.) an active, efficient process of entry into the target cell nucleus; iii.) relatively high levels of gene expression; iv.) the potential to target particular cellular subtypes through control of the vector-target cell binding and the tissue-specific control of expression; v.) a general lack of pre-existing host immunity; and vi.) substantial knowledge and clinical experience which has been gained with such vectors.

Presently, retroviruses are successfully used for ex vivo transduction in gene therapy experiments. However, the production technology for retroviral vectors is in an early state. Therapies are hampered by i) the inherent instability of infectious viral particles, ii) the difficulty to produce large amounts of virus, iii) the broad host range of currently available viral envelopes and the sensitivity to human complement attack. Thus both for ex vivo and in vivo applications, significant developments preclude a widespread application of retroviral gene therapy. The main hurdle is currently in the production of large number of stable viruses. The production process comprises the generation of virus, e. g. retroviral vectors, with virus producing cell lines.

Recombinant retroviruses for gene therapies transduce a retroviral vector encoding the effector (therapeutic) gene or therapeutic nucleotide sequence. Presently, they are produced after transfection of the vector into packaging cell lines stably expressing the viral proteins gag/pol and env. This principle applies for the classical vectors based on Moloney Murine Leukaemia Viruses (MLV). Lentiviruses are mostly produced from transient expression systems since the toxicity of helper genes, in particular the VSV-G protein (see below) requires a timely restricted expression.

Construction of packaging cells for retroviral vectors is a tedious procedure. Sequential transfection of appropriate gag/pol and env expression constructs and time-consuming screening procedures for a balanced and high-level expression of all components are needed. Heterogeneity in expression by individual cell clones is due to i.) random integration of the helper genes into the chromosomal DNA of the host cell and ii.) variable copy numbers of the transfected constructs. Presently, subcloning and testing of individual subclones is applied to isolate suitable packaging cells.

The outer shell proteins (env) are ligands for cellular receptors, mediate viral entry and define the host range of the virus. Retroviruses allow substitution of their envs and mixed retroviral particles are derived by this technique named pseudotyping For this purpose env proteins are used that have been isolated from naturally occurring non-murine viruses e.g. GALV, Gibbon Ape Leukaemia Virus and VSV G (Vesicular Stomatitis Virus, protein G). In addition, a large number of new env proteins have been created by recombinant techniques mainly with the emphasis of specifying the host range thereby achieving targeting of the virus to specific cell types, and conferring resistance to human complement. Further developments on env proteins promise improved therapies. This is also important in view of the known inherent retrovirus instability which is to a large degree due to the env proteins. The instability of the retrovirus during the production process, storage or during purification procedures is a major limitation for most applications. Presently, the only way to significantly improve retroviral stability is by replacing the env protein with the VSV G protein, resulting in particles that can be concentrated and stored for an extended time period. Despite its advantage in using VSV G for retrovirus pseudotyping, VSV G expression is toxic to producer cells. To date VSV G pseudotyped virus particles are produced in transient transfection systems or by inducible expression of the VSV G protein, thus, limiting the applicability of the system.

The use of the novel env proteins for therapeutic purposes is currently hampered by the fact that for each of the different env proteins, an individual packaging cell line has to be constructed. Each of these cell lines again has to be established according to the above described procedures since more economic methods have not yet been developed.

The above described problems apply mutatis mutandis to other viral system, like the lentivirus system, the adenovirus and the adenovirus associated virus system.

Thus, for efficient retrovirus production, viral helper genes (gagpol and env) as well as a therapeutic vector must be expressed in a so-called "balanced" ratio in producer cell lines. According to the state of the art, packaging cell lines as the precursors are established by random integration of the viral helper genes into the host cell genome. Since the integration site dramatically influences recombinant gene expression and its stability, intensive screening is required to isolate optimal packaging cell clones. This procedure has to be followed independently for any env gene. Even once this packaging cell line is established, a second round of screening has to be performed for preparation of the therapeutic virus particles. For establishment of packaging cells producing therapeutic retroviruses, i.e. virus producing cell lines, the packaging cells have to be transfected with a therapeutic vector. The nature of the randomly chosen integration site as well as the copy number affects the resulting virus titer, again necessitating another round of time-consuming screening for efficient and stable vector expression. Obviously, this screening has to be repeated for any therapeutic virus of interest.

In particular, the laborious and time-consuming screening procedure comprises the following steps:
(i) transfection of the vector and selection of transfected single cell clones
ii.) evaluation of the transgene expression
iii.) evaluation of the recombinant virus titer
iiii.) evaluation of the stability of expression and titer over longer cultivation periods
iiv.) characterization of the chromosomal integration site and determination of vector copy numbers
iv.) test for absence of RCRs (replication competent retroviruses)

Moreover, it was found that each virus producer cell clone needs further adaption for optimal production conditions. This work additionally covers a considerable amount of time and labour with regard to the overall production process.

To facilitate this screening in most cases the therapeutic viruses additionally carry a selection or marker gene. This marker gene allows selecting for stable integration and/or for high level, long term expression. However, in the last years evidence has been arising that the transduction of additional genes can impose significant problems in therapeutical settings. One of the major problems is that an immune response against the foreign genes (e.g. selection markers) is provoked. This can result in efficient elimination of transduced cells, thereby limiting or exclude the therapeutic success. Even more concern is raised since insertional leukemogenesis was observed in the mouse model due to the transduction of a marker gene that was prior considered to be biologically inert. Thus, the overall aim to improve and to make viral gene therapy safer is to exclude any coding sequences beside the therapeutic gene of interest.

In view of the above outlined still existing problems a broad application of gene delivery methods using virus is currently not possible.

### Summary of the present invention

The present invention provides new methods for the production of virus producing cell lines. In particular, the present invention aims at providing an improved system for preparing virus that may be of subsequent use in medicine.

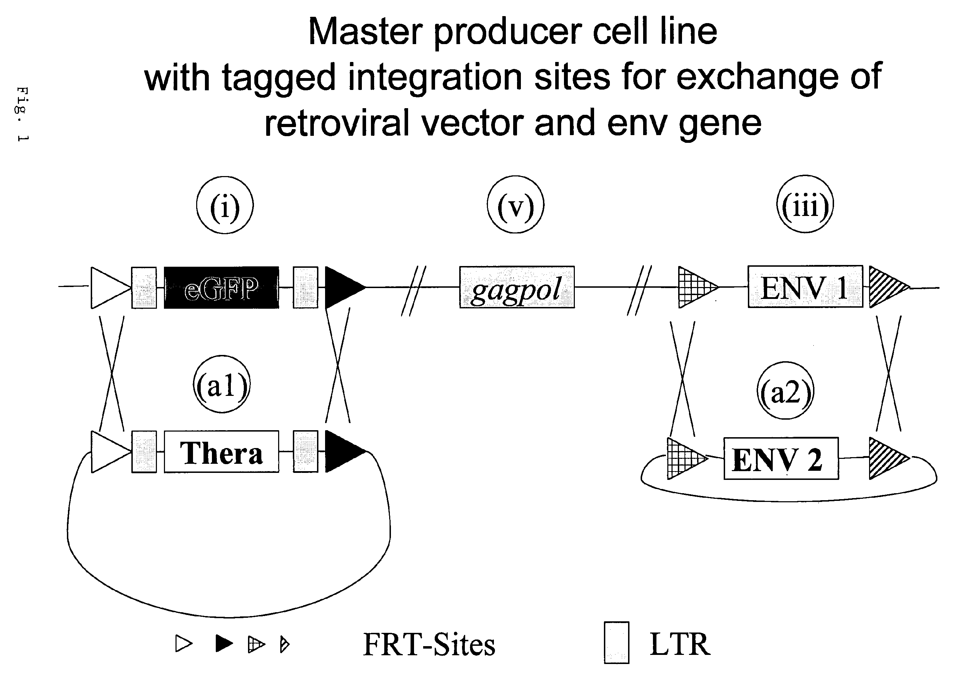

In particular, the present invention provides a method for the generation of virus producing cell lines using a master cell line once produced before. An example of this master cell line is depicted schematically in Fig.1. Said cell line allows for easy, specific and predictable integration of expression cassettes containing the nucleotides or gene of interest (Fig.1 (a1) and/or the envelope moieties (Fig.1 (a2) necessary to produce the desired type of virus. Due to the predicted integration site, the titer, the cultivation conditions and the safety record is not affected.

When using the master cell line no additional laborious screening of the cells is necessary after targeting the expression cassette of interest into the already tagged chromosomal loci in the master cell line. Thus, allowing simple exchange of various expression cassettes while maintaining the properties of the master cell line which was once selected for high expression chromosomal integration site allowing high and predictable virus production under known culture conditions.

The present invention further relates to the virus producing cell line obtainable with the method according to the present invention. The virus producing cell line according to the present invention allow for standardised production of virus which may be eventually used in gene therapy.

### Brief description of the figures

### Figure 1:

Master producer cell line with tagged integration sites for exchange of retroviral vector and env gene.

This overview exemplies the outline of the generation of therapeutic virus producer cell lines. In a first step, a retroviral tagging vector and the helper functions gagpol and env are stably integrated into the host's genome. Both the retroviral vector and the env gene are flanked by FRT sites, thereby allowing for site-specific replacement of the expression cassettes by a therapeutic vector and/or an alternative env gene.

### Figure 2:

Generation of a master producer cell line with one exchangeable cassette
A: A retroviral tagging vector was established that carries a reporter gene (GFP), an EMCV IRES element and a hygromycin resistance gene (EMCV element and hygromycin resistance gene are not shown for better overview). In the 3' U3 region of the LTR a tagging cassette was integrated comprising two non-interacting FRT-sites (represented by the triangles) and a start codon deficient and thus non-functional neomycin phosphotransferase gene (upper lane). This vector was stably transduced into PG13 packaging cells. For tagging, 293 cells were infected with the virus supernatant using a multiplicity of infection below 0,01 to ensure single copy integration. In infected cells, the retroviral vector is stably integrated according to the lower lane. As a consequence of reverse transcription, the 3'U3 region including the tagging cassette is duplicated to the 5' end, resulting in the depicted proviral state.
B: Infected, tagged cells were analyzed according to their GFP expression. The figure shows an overlay histogram of a flow cytometry analysis of a tagged cell clone (thick line) compared to non-infected cells (thin line).
C: The tagged cell clone 1 B2 was transfected in subsequent steps with the viral helper functions, i.e. the gagpol expression vector pCeB (Cosset et al., J. Virol. 69:7430-7436, 1995) and pENVA-his (Spitzer et al., J. virol 77, 6070-6075, 2003), an amphotropic env expression vector. Cells were selected for stable integration. Cells were screened for balanced expression of helper function gagpol and env, resulting in a high retroviral titer. The figure shows the efficiency of release of GFP transducing retroviruses of individual subclones (1 B2-8-F, IIIC2, and A-E) as compared to the tagged cell line 1 B2.

### Figure 3:

### Generation of a therapeutic virus producing cell line

A: The master cell clone 1 B2-8-F was targeted according to the depicted scheme. A targeting vector was constructed in which the therapeutic CollagenVII transducing retrovirus vector was flanked with the non-interacting FRT sites and further carries an ATG codon and an additional element ('P') to initiate neo expression upon targeting. Element 'P' can be either an independent promoter or e.g. an IRES element. In the latter case, neoR is obtained from an LTR derived RNA.
B. G418 resistant cell clones obtained upon targeting the retroviral vector cassette were analyzed for GFP expression. The histogram overlay shows the G418 resistant clones in comparison to the parental 1 B2-8-F clone and 293 cells as a control.
C: Correct targeting was confirmed by Southern blotting of G418 resistant clones. For this purpose, chromosomal DNA of parental master cell line 1 B2-8-F and IIIC2 and independent subclones was digested with Sacl and hybridyzed against an EMCV probe. Correct targeting results in a 2 kb fragment in correctly targeted clones and elimination of the 4 kb parental signal. Such analysis was conducted for about 50 independently raised subclones, over 95% of them showing correct targeted exchange (not shown).
D: Targeted cells were analyzed according to the expression of ColVII. For this purpose cells were immunostained using an antibody against hColVII.

### Figure 4:

A: Virus production was monitored. Mouse NIH3T3 cells were infected with the virus supernatant of a G418 resistant subclone. Infected and control cells were immunostained for hColVll.
B: The virus titer was monitored after targeting an eGFP transducing retroviral vector into 1 B2-8-F cells. G418 resistant clones were isolated, targeted exchange was confirmed by Southern blotting (not shown) and analyzed for their virus production. The figure B shows the uniform titer of subclones generated upon transfection according to the protocol described in the example.
C: The retroviral titer of a single G418 resistant targeted subclone was monitored over time. Fig. C shows the stability of the titer over 4 weeks.

### Detailed description of the invention

It is noted that as used herein the following terms have the meaning as indicated below:
The term "nucleotide of interest, NOI" as used herein is intended to mean a nucleic acid molecule which can include eukaryotic mRNA, cDNA from eukaryotic mRNA, genomic sequences from eukaryotic DNA and synthetic DNA or RNA sequences. Preferably the NOI is a nucleic acid sequence derived from a human DNA or RNA sequence. For example, the NOI encodes a molecule selected from the group consisting of proteins, peptides, antisense nucleic acid sequences, ribozymes and siRNA.
The term "operably linked" as used herein is intended to mean arrangements of elements wherein the components so described are configured so as to perform their usual function. Thus, a given promotor operably linked to a coding sequence is capable of effecting the expression of the coding sequence when the proper enzyme is present.
The term "virus" is interchangeably used with the term "virus particle" in the present application.
The term "virus producing cell" as used herein is intended to mean a cell which contains all elements necessary for the production of virus or virus particle. The term "cell line" refers to a population of isogenic cells capable of continuous growth and division in vitro.
The term "master producer cell (line)" as used herein is intended to mean a cell or cell line which contain chromosomally integrated at least a sequence encoding gag/pol, an expression cassette containing sequences allowing site specific integration, sequence(s) for a selection marker and a non-functional selection domain, and a different expression cassette that preferably but does not necessarily contain sequences allowing site specific integration, sequence(s) for a selection marker different to the selection marker in the other expression cassette and a non-functional selection domain.
The term "selection marker" as used herein is intended to mean a marker which enables discrimination of moieties, like cells, which may or may not contain said selection marker. For example, a selection marker may be the expression of a specific molecule, a resistance marker, like a marker conferring drug-resistance to a cell, etc. Thus, the selection marker allows for simpler preparation, manufacturing, characterization or testing of the cell or cell line.
The term "functional selection domain" as used herein is intended to mean a domain being functional in that it contains an element allowing the selection of cells. The functional selection domain comprises at least two non-functional selection fragments.
Non-functional selection fragments means that the selection element is not present in a functional form and, hence, can not be used for selecting purposes between different cells. "Non-functional selection fragments" are fragments which do not contain all elements necessary to allow the selection based on the selection element. The combination of at least two complementing non-functional selection fragments results in a functional selection domain. It can be a resistance marker or another marker that allows for characterization of the cells. Further, the non-functional selection fragment may be a silent but functional selection marker. That is, the functional selection marker is not transcribed due to a missing promoter or missing enhancer or missing translation initiator sequences.
The term "site-specific integration" as used herein is intended to mean that integration of elements, like expression cassettes, is possible at predefined sites only.
The term "tagged" as used herein is intended to mean a state in which a chromosomal site is genetically marked/labelled by integration of a nucleotide sequence that allows for site specific integration and subsequent selection
The term "targeted" as used herein is intended to mean the state a nucleotide sequence has been site specifically integrated into a previously tagged chromosomal integration site.

The present invention relates to a method for the generation of a virus producing cell line comprising the following steps:
a1.) introducing into a master producer cell line a nucleotide construct comprising a nucleotide of interest (NOI) cassette comprising sequence(s) of nucleotide(s) of interest (NOI) operably linked to a promoter having long terminal repeats (LTR) upstream and downstream of the NOI sequence, respectively, a non-functional selection fragment la allowing the formation of a functional selection domain I when combined with the complementary fragment Ib present in the master producer cell line downstream of the 3' LTR; and the NOI cassette being flanked on both sites by sequences allowing site-specific integration of said NOI-cassette into an expression cassette present in said master producer cell line; and/or
a2.) introducing into the master producer cell line a nucleotide construct comprising an env expression cassette comprising sequence(s) of env gene(s) operably linked to a promoter, a non-functional selection fragment lla allowing the formation of a functional selection domain II, which may be identical to or different from the functional selection domain I, when combined with the complementary fragment IIb present in the master producer cell line downstream of the sequence(s) of env gene(s), and said cassette being flanked on both sites by sequences allowing site-specific integration of said env expression cassette into an expression cassette present in said master producer cell line;
b.) initiating replacement of the NOI-cassette and/or env-cassette with the corresponding expression cassette present in the master producer cell line;
c.) selecting cell lines having stably integrated the NOI-cassette and/or the env-cassette on the basis of the functional selection domain(s).
with the proviso that once before the master producer cell line used in a.) is obtained by
(i) stably integrating an expression cassette into the genome of a host cell or cell line, said expression cassette comprises a nucleotide construct carrying at the 5' site a sequence allowing for site-specific integration, a promoter, operably linked with a sequence encoding a selection marker I, and at the 3' site a sequence allowing for site-specific integration being different to the sequence present at the 5'-site, the sequence at 3' site further contains downstream a non-functional selection fragment Ib;
(ii) selecting a transduced cell line wherein the expression cassette of (i) is stably integrated into the host genome resulting in a transduced cell line tagged with the expression cassette of (i);
(iii) stably integrating a different expression cassette into the genome of the cell line, said expression cassette comprises a nucleotide construct carrying optionally at the 5' site a sequence allowing for site-specific integration, a promoter, operably linked with a sequence encoding a selection marker II being different from selection marker I, and optionally at the 3' site a sequence allowing for site-specific integration being different to the sequence present at the 5'-site, downstream of the optional sequence at the 3' site a non-functional selection fragment IIb which may be the same or may be different to selection fragment Ib is present, the optional sequences allowing site-specific integration may be identical with the sequences flanking the expression cassette of (i);
(iv) selecting a transduced cell line wherein the expression cassette of (iii) is stably integrated into the host genome resulting in a transduced cell line tagged with the expression cassette of (iii);
(v) stably integrating a different expression cassette into the genome of the cell line, said expression cassette comprises a nucleotide construct containing a promoter operably linked to a sequence encoding gag/pol;
(vi) selecting a transduced cell line wherein the expression cassette of (v) is stably integrated into the host genome resulting in a transduced cell line tagged with the expression cassette of (v);
and wherein the order of the integration of the different expression cassettes according to (i), (iii) or (v) is not determined.

In a first step, a master producer cell line is produced according to steps (i) to (vi). Of course, when starting the method with a cell or cell line containing already one of the elements to be introduced into the cell, only the remaining steps have to be performed.

In addition, the integration of the different cassettes according to (i), (iii) and (v) may be conducted at the same time. In particular, the integration of the gag/pol and another expression cassette may be performed simultaneously.

With the first stage a "master producer cell line" is produced. This master producer cell line is generated once providing a tagged cell line having incorporated into its genome a gag/pol expression cassette and two expression cassettes each containing a selection marker. Thus, the master producer cell line represents a cell line selected for optimized expression of the various expression cassettes. The selection steps have to be performed only once for each expression cassette tagged into the cell line.

In the second stage according to the method of the present invention, the master cell line is targeted with the NOI and/or the env gene of interest.

That is, the second stage comprises a gene cassette replacement wherein the replacement of the gene cassette takes place at the previously tagged sites, the sequences of site specific integration, in the genome of the master producer cell line. Thus, the cassette(s) are integrated into the tagged chromosomal loci of the master producer cell line. The integration of the cassette(s) of interest into the particular known sites results in virus producing cell lines having an optimal expression of the targeted cassettes. In addition, since the replacement takes place at the sites that allow site specific integration in the host genome, no laborious selection steps are necessary. In particular, the introduced expression cassette(s) will not integrate randomly into the host genome but specifically into the tagged chromosomal loci having the site specific integration sites. Consequently, in the final virus producer cell line the chromosomal sites into which the helper components and the retroviral vector are integrated are identical to the corresponding sites of the master producer cell line. Thus, the final virus producer cell line will have exactly the same properties as the master producer cell line, in particular in view of the efficiency of virus production, fermentation conditions and safety features.

Moreover, the presence of the LTR sequences in the NOI cassette allows for the production of a recombinant viral genome which does not contain any additional sequences, e.g. sequences encoding a selection marker, like resistance to antibiotics, etc. Thus, the virus or virus particle produced according to the method of the present invention is free of additional components which may adversely affect the therapeutical benefits of the NOI. It is within the scope of the present invention that the LTR sequences may be substituted with sequences having the same function, i.e. enabling the generation of viral vector containing the NOI.

In more detail, the first stage resulting in a master producer cell line comprises the steps of tagging chromosomal integration sites by integrating the different expression cassettes into the host genome and selecting appropriate cells. At least one of the different expression cassettes introduced into the genome comprise sequences at the 5' -site of the cassette allowing for site-specific integration, further, an additional promoter is present downstream of the above mentioned 5'-site. The additional promotor is operably linked with a sequence encoding a selection marker I or II, respectively. Selection markers I and II are different from each other. At least one of the cassettes having a site-specific integration site at the 5' end also contains at the 3' -site a second sequence allowing for site-specific integration. Further downstream a non-functional selection fragment Ib or llb, respectively is present. The site specific integration sites at the 5' end and the 3' end are different from each other.

With "sequences allowing site specific integration" sequences are meant which allow for a replacement exchange of the cassette integrated into the host genome, the tagged cassette, with a different cassette, the targeting cassette, which should be integrated into the genome by replacement of the integrated tagged cassette. Preferably, a recombinase assisted mechanism is used for the replacement exchange. This system facilitates the exchange. From the recombinase systems known the Cre recombinase/ loxP recognition sites of bacteriophage P1 or the site-specific FLP recombinase of S. cerevisiae are the most frequently used.

The method conducted in the first stage to establish a transduced cell line or master producer cell line is well known in the art. The selection is based on various expression characteristics like the expression of a selection marker, introduced within the tagging cassette. Additional criteria for the selection of an appropriate cell line are an optimal expression level that is stable over time, the maintenance of fermentation conditions, the cell growth, the genomic stability of the integration site and safety features.

The order of the integration of the expression cassettes according to steps (i), (iii) and (v) is not determined. That means, one may start with the integration of the gag/pol cassette first. Moreover, if cell lines are already present having at least one of the cassettes of steps (i), (iii) and (v) stably integrated in their genome, said cell lines may be used for further integration of the cassettes not yet integrated in the genome. Further, at least two cassettes may be integrated simultaneously. In particular, it is possible to introduce the gag/pol and another expression cassette at the same time for integration into the cell genome.

The expression cassette to be integrated in steps (i) and/or (iii) comprises at least the following elements:
Site-specific integration elements at the 5' and 3' end of the selection marker sequence. These elements include recognition sites for enzymes which enable specific exchange for sequences flanked by the same recognition sites into the specific chromosomally integrated nucleotide sequence. A typical example of this system is the yeast Flp recombinase system. To avoid the deletion of the integrated product at a later time point, it is preferred to use two non-interacting recombination sites at the 5' end and 3' end.
An promoter and/or another element (e.g. an IRES element) for enabling transcription and/or translation of the selection marker.
A selection marker. The selection marker may be a drug-resistance marker like neomycin phosphotransferase, an expression marker within the cell (e.g. gfp, ß-galactosidase) or at its surface (e.g. receptors) or it may be an env protein itself.
A non-functional selection fragment. This non-functional selection fragment represents an incomplete selection marker, e.g. a drug resistance marker. Since the fragment represents an incomplete domain, selection on the basis of the selection marker is not possible, but it requires completion with at least one additional non-functional selection fragment. This additional fragment is supplied later by the integration of the NOI and/or env cassette according to steps a1) and/or a2) respectively, thus, only specific and precise integration in step b) render the cell in a position to be selectable on the basis of the selection marker.

As indicated above, the sequences allowing site specific integration at the 5' and 3' site of the cassettes are preferably non-interacting. Using different sequences avoids unwanted excision of the insert. Moreover, the sequences for site specific integration may be identical or may be different in the cassettes of step (i) and (iii). Preferably, said sequences are different from each other. Thus, specific integration or exchange of only one cassette is conducted.

The selection process for selecting transduced cells having chromosomally integrated the desired cassette is conducted according to well known techniques taking the desired characteristic of the transduced cells into account, like high-level production, stability and safety.

The first stage of the method according to the present invention results in a transduced cell line or master producer cell line having chromosomally integrated usually at least three cassettes. The integration sites are also referred to as the tagged chromosomal loci. This cell line is selected for the desired parameters, like stability of the integrated cassettes etc. When using the env protein as one of the selection markers I or II, the resulting cells have all genetic elements required for recombinant virus production. In addition, if a sequence encoding the env protein is used for the generation of the master cell line, wherein the sequence encoding the env protein represents the sequence encoding either selection marker I or II, the tagged cassette does not necessarily contain sequences at the 5' and 3' end allowing site-specific integration.

In the second stage the virus producing cell line is established. By using site-specific integration sites being identical to the site specific integration sites present in the chromosomally integrated cassettes, it is possible to specifically replace the cassette present in the master producer cell line with the NOI and/or env cassette.

The use of the master producer cell line allows for a site specific integration efficiency of more than 95%. In addition, the expression characteristics are maintained after the exchange of the tagged selection marker introduced in the first stage with the NOI and/or env cassette. Moreover, different clones obtained in the second stage of the method according to the present invention demonstrate a titer that is stable and predictable in all subclones.

The NOI cassette or the env cassette introduced in the second stage of the method according to the present invention comprises the site-specific integration sites at the 5' and 3' side flanking the cassettes. These site specific integration sites are identical to at least one of the site specific integration sites present in the expression cassettes which are chromosomally integrated into the master producer cell line. Moreover, the cassettes introduced during the second stage contain a non-functional selection fragment which allows the formation of a functional selection domain when combined with the complementary fragment present in the tagged chromosomal loci.

The formation of a functional selection domain provides for an extremely effective selection procedure for correct cassette replacement. Thus, laborious selection work is avoided but only cells wherein the correct cassette replacement took place can be selected on the basis of the functional selection domain.

The functional selection domain contains a selection marker, like a drug-resistance selection marker, antibiotics etc. For example, the tagged chromosomal locus of the master producer cell line encompasses the sequence for encoding drug-resistance except for the initiation codon or the promotor sequence. By supplementing the fragment with the non-functional selection fragment present in the targeting cassette (NOI and/or env cassette), the missing in frame initiation codon or the promoter is introduced, thus, providing a functional selection domain which can be used for selection purposes.

The above described system overcomes the laborious and time-consuming work for selection suitable clones. Moreover, the method according to the present invention enables the use of the master producer cell line for the production of a variety of different cell lines for the production of virus having different env proteins or containing viral vector encoding different nucleotides of interest, e.g. different therapeutic genes etc. allowing the expression of therapeutically useful proteins in the target cell.

Moreover, simple and fast pseudotyping of the virus is possible. The term pseudotyping means incorporating in at least a part of, or substituting at least a part of, or replacing all of, an env gene of a viral genome with a heterologous env gene, for example an env gene from another virus. Pseudotyping can improve viral vector stability and transduction efficiency and specificity.

If necessary, the cassettes introduced into the cell or cell line contain a promotor which is operably linked with the selection marker or the NOi or the env sequence. The promotor may be selected from known promotor sequences including constitutive promoters (e.g. CMV, and promoters for timely restricted (controlled) expression (e.g. Tet-controllable, tTA dependent promoters).

However, an additional promotor besides the LTR sequences is not necessary, since the LTR itself represents a structure promoting the transcription of the sequences downstream of the LTR sequence.

Thus, the NOI cassette additionally comprises all genetic cis-elements required for proper packaging of the RNA, reverse transcription and integration, e.g. flanking long terminal repeat (LTR). The LTRs are responsible for proviral integration, and transcription. They also serve as enhancer-promoter sequences. That means, the LTRs can control the expression of the recombinant therapeutic genes. Flanking of the NOI sequence by the LTRs enables the production of viral vectors which do not contain any additional sequence encoding a molecule which may adversely affect the therapeutic effectiveness of the virus. In particular, the viral vector does not contain any sequence encoding for a selection marker or other molecules necessary during the production process of the virus.

In a preferred method according to the present invention, the expression cassettes of steps (i) and (iii) are transduced by retroviral gene transfer and therefore contain 5' LTR and 3' LTR. In the U3 region of the 3' LTR the non-functional selection fragment may be integrated. Further, the 3' LTR contains the site-specific integration sites. As a consequence of the process of retroviral infection the insert of the 3'U3 insert is duplicated to the U3 of the 5'LTR, thereby flanking the expression cassettes with site-secific integration sites.

Alternatively, the introduction of the cassettes to be integrated into the cells is performed by non-retroviral transfection methods which are well known to the skilled artisan. For example, the introduction of the cassettes may comprise infection with viral vectors, transfection with liposomes also called lipofection, transfection using the well known calciumphosphate technique or electroporation. Transfection generally refers to the uptake of foreign DNA by a cell.

The replacement of the expression cassettes of steps (i) and (iii) is conducted for example with the help of a recombinase or a restriction enzyme. That is, the enzyme necessary to promote or catalyse the exchange is for example is the Cre recombinase or the FLP recombinase or any other recombinase known in the art. Alternatively, the exchange may be promoted by using specific restriction enzymes like SCEI.

The genes, encoding the above mentioned enzymes may be introduced into the cell by transfection or infection of appropriate expression vectors. The transfection may be a stable or a transient transfection. Moreover, the recombinase system may be an inducible system. Alternatively, the enzyme as such may be directly provided into the cell culture system.

The selection of cell lines having stably integrated the NOI-cassette and/or the env cassette is conducted on the basis of the expression of the selection markers. In particular, the supply of one of the necessary non-functional selection fragments to complete the non-functional selection fragment in the tagged chromosomal locus provides for a selection marker, e.g. drug-resistance marker gene having functional activity only in cells where an exchange of cassettes took place. Thus, the selection is an easy and reliable process overcoming the laborious and time consuming work which is necessary to select suitable clones.

Further, since the process maintains and does not affect the structure of the chromosomal integration sites, the expression of the NOI will have the same expression characteristics as the marker gene in the master producer cell line.

The present invention further relates to the virus producing cell line itself. The virus producing cell according to the present invention affords the production of virus or virus particle containing a viral vector. Importantly, this vector does not need to contain any coding sequence which may adversely affect the therapeutic benefits of the NOI. By providing 5' LTR and 3' LTR upstream and downstream of the NOI sequence, respectively, together with the known cis acting elements, psi and the polypurine tract required for packaging and reverse transcription, it is possible to obtain a viral vector containing only the NOI sequence. This viral vector is packaged into the virus which may subsequently be used to infect the target cell.

Within the scope of the present invention, there is the virus obtainable with the claimed method. The virus is characterized in having a viral vector which does not contain any additional sequence which product may adversely affect the therapeutic benefits of the NOI present in the viral vector. In particular, the virus according to the present invention does not need to encode for a selection marker being used during the production process for the generation of the virus.

Thus, the virus according to the present invention is particularly useful in gene therapy. Hence, the present invention relates also to methods for treating patients using the virus according to the present invention. Moreover, the present invention relates to a therapeutic or pharmaceutical for use in gene therapy. The skilled person is well aware of the composition containing the virus as an active ingredient.

Preferably, the virus is a retrovirus, for example mouse retroviruses or lentiviruses. In particular retrovirus include: mammalian C-type viruses like Moloney murine leukaemia virus (Mo-MLV), Moloney sarcoma virus (Mo-MSV), Abelson murine leukaemia virus (A-MLV), mammalian B-type viruses like mouse mammary tumour virus (MMTV), avian leukosis-sarcoma virus like Rous sarcoma virus (RSV) and Fujinami sarcoma virus (FuSV), lentiviruses like human immunodeficiency virus (HIV) and simian immunoddeficiency virus (SIV), equine infectious anaemia virus (EIAV), Spumavirus and human T-cell leukemia virus HTLV.

The env introduced into the master producer cell line to arrive at a virus producing cell line according to the present invention, may be the env obtained from the same virus as the gag pol proteins. Alternatively, when pseudotyping the virus, an env protein from a heterologous virus may be used. Pseudotyping may improve viral stability and transduction efficiency, specificity of transduction as explained above. The env proteins to be used in the method according to the present invention may include env 10A1, ecotropic env, GALV, RD114, LMCV, 4070A genetically modified envs e.g. that confer stability to complement, or target specific cell types, and chimeric envs.

In addition, master producer cell lines producing toxic or harmful proteins like VSV-G can be established using expression cassettes that provide a timely restricted (conditional) expression of the protein.

In the method according to the present invention the host cell used for the production of the master producer cell line may be selected from the group consisting of mammalian cells like HT108, HEK293, Tert-immortalized cell lines etc. The skilled person is well aware of suitable cell or cell lines usable for the production of the master producer cell line and the generation of the virus producing cell line according to the present invention.

The present invention also provides the use of a viral vector, in particular of a retroviral vector, of the invention in the manufacture of a pharmaceutical composition The pharmaceutical composition may be used to deliver a NOI to a target cell in need of the same. In particular, the pharmaceutical composition may be used in gene therapy. The composition may optionally contain a pharmaceutically acceptable carrier, diluent, excipient or adjuvant. The composition may additionally contain known pharmaceutical ingredients like binder, etc.

### Examples

Example 1 Generation of a master producer cell line

### Step a Tagging cells with a GFP reporter cassette

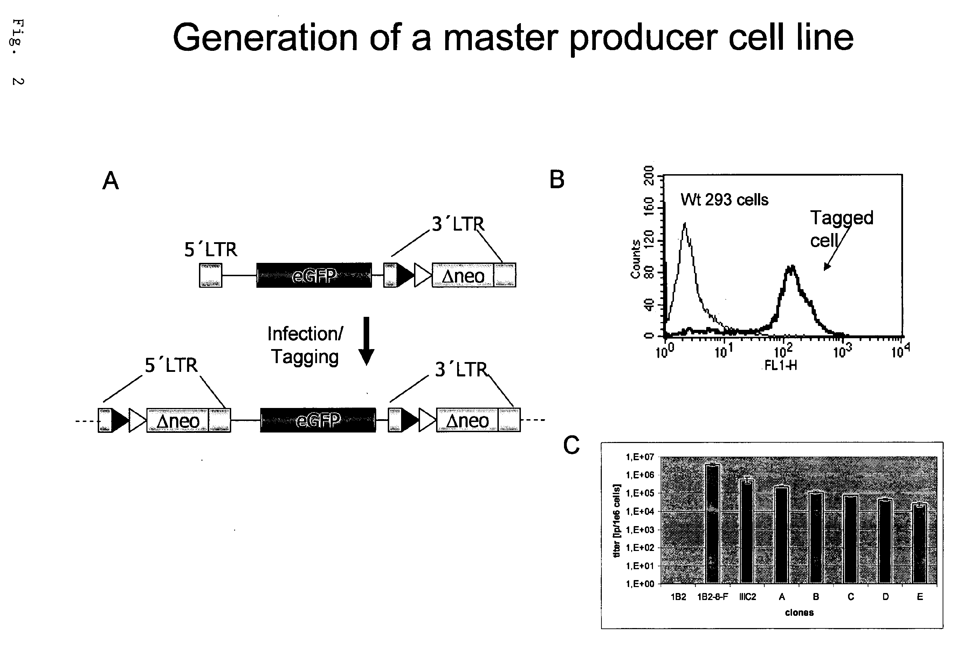

A retroviral tagging vector pROL was constructed. This vector contains MSCV derived LTRs and transduces a bicistronic expression cassette comprising GFP (green fluorescent protein) and hygromycin B phosphotransferase. In addition, in the 3'LTR (Nhel site) two non-interacting FRT sites and a sequence comprising an ATG-deficient, non-functional neomycin phosphotransferase gene was integrated (see Fig.2a, upper line for a schematic drawing of the vector). This vector (pROL) was stably transfected into the PG13 packaging cell line as described in Spitzer et al, Hum Gene Ther. 10, 1893-1902 (1999). Briefly, 5*µ*g of pROL was transfected to 1×105 cells. The cells were selected for Hygromycin resistance (200 U/ml). The GFP titer was determined. Subsequently, 1×105 HEK293 cells were infected in the presence of 8*µ*g/ml polybrene as described in Spitzer et al, Hum Gene Ther. 10, 1893-1902 (1999), thereby applying a multiplicity of infection below 0,01. Two days after infection, cells were selected for Hygromycin resistance. Hygromycin resistant cells with optimal GFP expression were isolated by Fluorescence activated cell sorting (FACS) (Fig. 2b).

### Step b integration of env and gag/pol

GFP high-expressing clones were checked for single copy integration by Southern blot analysis. Subsequently, single clones were sequentially cottransfected with the helper functions. For this purpose, 5 µg of the amphotropic env expression vector pENVA-his (Spitzer et al., J. virol 77, 6070-6075 (2003)) was transfected to 3x105 cells of the tagged cell clone. Cells were selected in medium containing 11 mM Histidinol and env expression was confirmed on isolated clones by flow cytometry. Then, pCeB, a gagpol expression vector conferring resistance to Blasticidin (Cosset, et al J. Virol. 69:7430-7436 (1995)), was transfected, again using 5µg of vector and 3x105 cells. Cell clones resistant to 3µg/ml Blasticidin were further characterized.

Clones were screened for maximal production of GFP transducing retroviruses (Fig. 2c). Single cell clones were monitored stable retrovirus production. In this example, the second expression construct (envA) is not exchangeable.

### Example 2 Generation of a virus producer cell line

### Exchange of the tagging cassette GFP with a collagen expression cassette

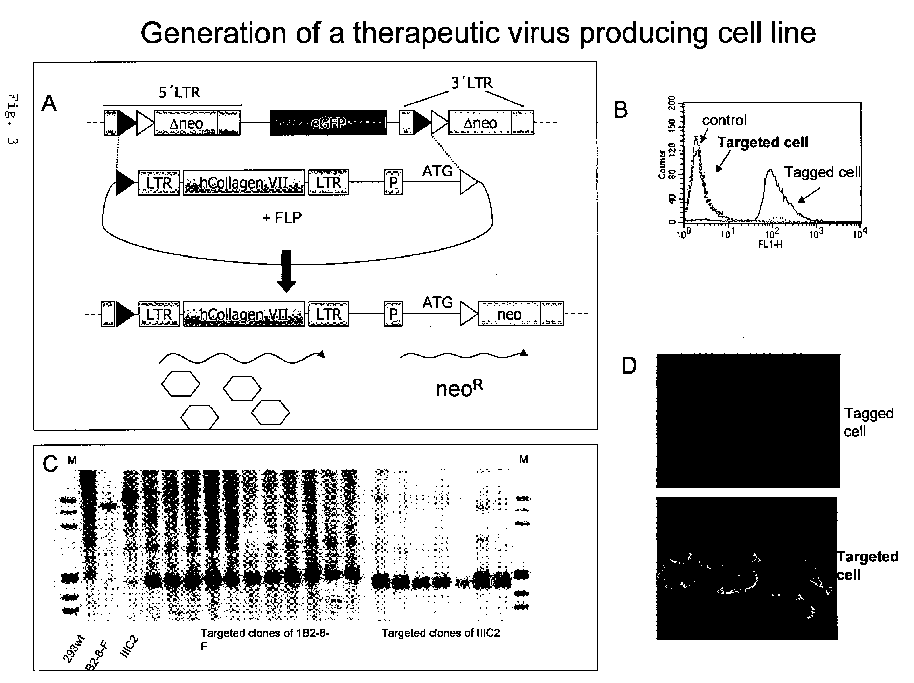

A therapeutic vector was integrated according to Fig. 3a. 2 µg of the targeting vector pTec4 (containing an MLV based retroviral vector transducing human ColVll cDNA) was cotransfected with 10µg of a Flp recombinase encoding vector pSVFlpe using DNA/Calcium phosphate coprecipitation on 3x105 HEK293 cells on 6 well plates as described in Spitzer et al, Hum Gene Ther. 10, 1893-1902 (1999). The medium was refreshed 6 hours post transfection. The cells were incubated for 4 days to allow the targeting process take place, and then 1*104 to 5*105 cells were seeded on 10 cm culture plates and set under selection pressure of G418 (1000µg/ml) for at least two weeks. Single cell clones were isolated and correct targeting was confirmed by GFP expression (Fig.3b) and Southern Blot analysis (Fig.3c). Immunostaining against ColVII showed a signal specifically in targeted cells (Fig 3d).

### Stable titer of cells with correctly targeted cells

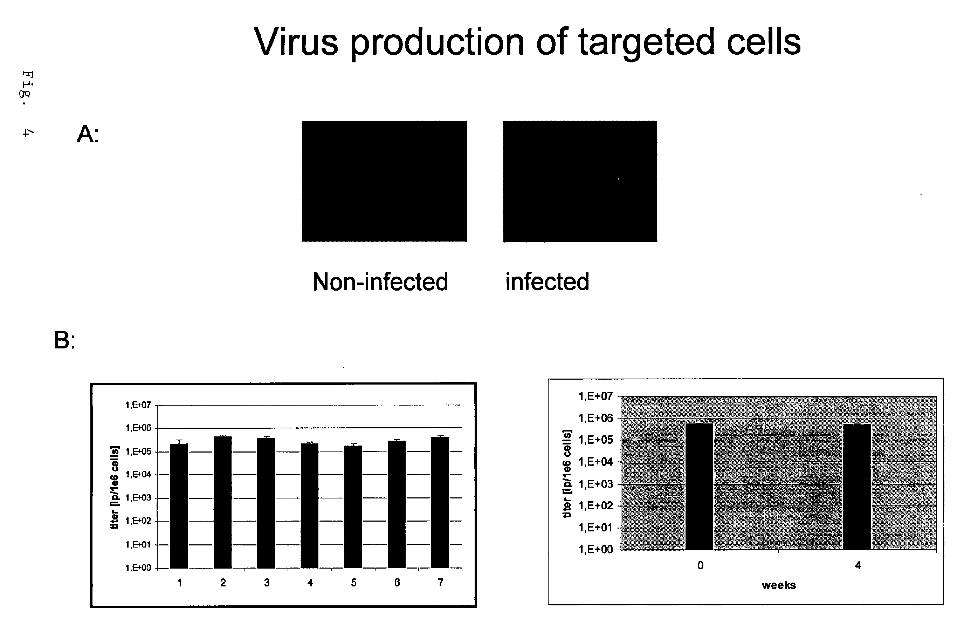

The virus supernatant of ColVII targeted cells was used to infect NIH3T3 cells in presence of 8µg/ml polybrene (Spitzer et al, Hum Gene Ther. 10, 1893-1902 (1999)). Expression of ColVII was documented by immunostaining using a ColVII antibody (Fig. 4a). Virus production after targeting was analyzed in parallel using cells being targeted with a vector in which the ColVll expression unit was exchanged for eGFP. Fig 4b shows the virus titer of independent cell clones obtained upon targeting and selection for G418 resistance. Importantly, all analyzed targeted subclones show a consistant (Fig 4b) and stable (Fig 4c) titer for at least 4 weeks proving that this method leads to predictable and stable virus production.

## Claims

1. Method for the generation of a virus producing cell line comprising the following steps:
a1.) introducing into a master producer cell line a nucleotide construct comprising a nucleotide of interest (NOI) cassette comprising sequence(s) of nucleotide(s) of interest operably linked to a promoter, having long terminal repeats (LTR)upstream and downstream of the NOI sequence, respectively, a non-functional selection fragment la allowing the formation of a functional selection domain I when combined with the complementary fragment Ib present in the master producer cell line downstream of the 3' LTR and the NOI cassette being flanked on both sites by sequences allowing site-specific integration of said NOI-cassette into an expression cassette present in said master producer cell line; and/or
a2.) introducing into the master producer cell line a nucleotide construct comprising an env expression cassette comprising sequence(s) of env gene(s) operably linked to a promoter, a non-functional selection fragment lla allowing the formation of a functional selection domain II, which may be identical to or different from the functional selection domain I, when combined with the complementary fragment IIb present in the master producer cell line, downstream of the sequence(s) of env gene(s) and said cassette being flanked on both sites by sequences allowing site-specific integration of said env expression cassette into an expression cassette present in said master producer cell line;
b.) initiating replacement of the NOI-cassette and/or env-cassette with the corresponding expression cassette present in the master producer cell line;
c.) selecting cell lines having stably integrated the NOI-cassette and/or the env-cassette on the basis of the functional selection domain(s).
with the proviso that once before the master producer cell line used in a.) is obtained by
(i) stably integrating an expression cassette into the genome of a host cell or cell line, said expression cassette comprises a nucleotide construct carrying at the 5 ' site a sequence allowing for site-specific integration, a promoter, operably linked with a sequence encoding a selection marker l, and at the 3' site a sequence allowing for site-specific integration being different to the sequence present at the 5'-site, the sequence at 3 ' site further contains downstream a non-functional selection fragment Ib;
(ii) selecting a transduced cell line wherein the expression cassette of (i) is stably integrated into the host genome resulting in a transduced cell line tagged with the expression cassette of (i);
(iii) stably integrating a different expression cassette into the genome of the cell line, said expression cassette comprises a nucleotide construct carrying optionally at the 5' site a sequence allowing for site-specific integration, a promoter, operably linked with a sequence encoding a selection marker II different from selection marker I, and optionally at the 3' site a sequence allowing for site-specific integration being different to the sequence present at the 5'-site, downstream of the optional sequence at the 3' site a non-functional selection fragment IIb which may be the same or may be different to selection fragment Ib is present, the optional sequences allowing site-specific integration may be identical with the sequences flanking the expression cassette of (i);
(iv) selecting a transduced cell line wherein the expression cassette of (iii) is stably integrated into the host genome resulting in a transduced cell line tagged with the expression cassette of (iii);
(v) stably integrating a different expression cassette into the genome of the transduced cell line, said expression cassette comprises a nucleotide construct containing a promoter operably linked to a sequence encoding gag/pol;
(vi) selecting a transduced cell line wherein the expression cassette of (v) is stably integrated into the host genome resulting in a transduced cell line tagged with the expression cassette of (v);
and wherein the order of the integration of the different expression cassettes according to (i), (iii) or (v) is not determined.

2. The method according to claim 1 wherein the expression cassettes of (i) and/or (iii) additionally contain a 5' and 3' LTR.

3. The method according to claim 1 or 2 wherein the introduction of the nucleotide construct(s) is performed by transfection or an infection.

4. The method according to any one of claims 1 to 3 wherein the integration of the cassettes is effected by a recombinase system and/or by restriction enzymes.

5. The method according to claim 4 wherein the recombinase system is the Cre recombinase/ loxP recognition sites of bacteriophage P1 or the site-specific FLP recombinase of S. cerevisiae.

6. The method according to claim 5 wherein the recombination system for site-specific integration is the FLP system using different FLP recognition target (FRT) variants wherein the sequences flanking the NOI-cassette and the env-cassette on both sites allowing site-specific recombination are non-interacting sequences being different from each other.

7. The method according to any one of the preceding claims wherein the non-functional selection fragment present in the NOI-cassette and/or the env-cassette contains a promoter or an IRES element operably liked to an initiation codon-sequence allowing the expression of a selection marker different to the selection marker I or II when complemented with the complementary non-functional selection fragment.

8. The method according to any one of the preceding claims wherein the non-functional selection fragment present in the NOI-cassette and/or the env-cassette contains only a promoter that activates a silent but functional selection marker integrated upon the tagging step.

9. The method according to any one of the preceding claims wherein the non-functional selection fragment present in the NOI-cassette and/or the env-cassette contains a splice signal that allows for translation of the selection marker

10. The method according to any one of the preceding claims wherein the NOI encodes a molecule selected from the group consisting of proteins, peptides, antisense nucleic acid sequences, ribozymes, and siRNA.

11. The method according to claim 10 wherein the NOI is a therapeutically useful molecule.

12. The method according to any one of the preceding claims wherein the selection markers I and/or II are selected from the group consisting of antibiotics, drug-resistance, cellular and extracellular expression markers likegfp, ß-galactosidase, secreted alkaline phosphatase, cell surface markers

13. The method according to any one of the preceding claims wherein the cassettes contain an additional promoter operably linked to the sequences to be transcribed including constitutive promoters (e.g. CMV, and promoters for timely restricted (controlled) expression (e.g. Tet-controllable, tTA dependent promoters).

14. The method according to any one of the preceding claims wherein the recombinase is introduced in the packaging cell by transfection or infection.

15. The method according to any one of claims 1 to 13 wherein the recombinase is introduced into the system by directly providing the enzyme to the cells.

16. The method according to any one of claims 1 to 13 wherein the gene encoding the recombinase is stably integrated into the host genome operably linked with an inducible promotor.

17. The method according to any one of the preceding claims wherein the virus is pseudotyped.

18. Virus producing cell line obtainable with a method according to any one of claims 1 to 17.

19. Master producer cell line obtainable with a method according to any one of claims 1 to 17.

20. Use of the virus producing cell line according to claim 18 or the master producer cell line according to claim 19 for the production of virus.

21. Virus obtainable with the method according to any one of claims 1 to 17.

22. Use of the virus according to claim 21 for the production of a therapeutic for gene therapy.
